(19) Europäisches Patentamt
European Patent Office
Office européen des brevets

(11) EP 1 131 273 B1

(12) **EUROPEAN PATENT SPECIFICATION**

(45) Date of publication and mention of the grant of the patent:
**22.10.2003 Bulletin 2003/43**

(21) Application number: **99958849.4**

(22) Date of filing: **09.11.1999**

(51) Int Cl.[7]: **C07C 33/48**, C07C 43/295

(86) International application number:
**PCT/US99/26434**

(87) International publication number:
**WO 00/029362 (25.05.2000 Gazette 2000/21)**

(54) **1,4-DIARYL-2-FLUORO-1-BUTEN-3-OL COMPOUNDS AND THEIR USE IN THE PREPARATION OF 1,4-DIARYL-2-FLUORO-1,3-BUTADIENE AND 1,4-DIARYL-2-FLUORO-2-BUTENE COMPOUNDS**

1,4-DIARYL-2-FLUOR-1-BUTEN-3-OL-VERBINDUNGEN UND IHRE VERWENDUNG IN DER HERSTELLUNG VON 1,4-DIARYL-2-FLUOR-1,3-BUTADIEN- UND 1,4-DIARYL-2-FLUOR-2-BUTEN-VERBINDUNGEN

COMPOSES 1,4-DIARYL-2-FLUORO-1-BUTENE-3-OL ET LEUR UTILISATION POUR PREPARER DES COMPOSES 1,4-DIARYL-2-FLUORO-1,3-BUTADIENE ET DES COMPOSES 1,4-DIARYL-2-FLUORO-2-BUTENE

(84) Designated Contracting States:
**AT BE CH CY DE DK ES FI FR GB GR IE IT LI LU MC NL PT SE**

(30) Priority: **16.11.1998 US 192680**

(43) Date of publication of application:
**12.09.2001 Bulletin 2001/37**

(73) Proprietor: **BASF AKTIENGESELLSCHAFT
67056 Ludwigshafen (DE)**

(72) Inventors:
- **HU, Yulin
Plainsboro, NJ 08536 (US)**
- **HUNT, David, Allen
Greenville, NC 27858 (US)**

(74) Representative: **Pohl, Michael, Dr. et al
Reitstötter, Kinzebach & Partner (GbR)
Patentanwälte
Postfach 21 11 60
67011 Ludwigshafen (DE)**

(56) References cited:
**EP-A- 0 811 593** **EP-A- 0 811 596**

- **B.M. MIKHAILOV, ET AL.: "Polyenic compounds. Communication 2. Polyenic compounds containing heterocyclic substituents" BULLETIN OF THE ACADEMY OF SCIENCES OF THE USSR, DIVISION OF CHEMICAL SCIENCE, no. 7, July 1956 (1956-07), pages 1489-1492, XP000879444 Consultants Bureau, New York, US ISSN: 0568-5230**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 131 273 B1

## Description

1,4-Diaryl-2-fluoro-1,3-butadiene compounds, methods for their preparation, and their use as intermediates in the preparation of 1,4-diaryl-2-fluoro-2-butene insecticidal and acaricidal agents are described in EP 811593-A1. The methods described in EP 811593-A1 for the preparation of 1,4-diaryl-2-fluoro-1,3-butadiene compounds require the use of phosphonium halide compounds. However, these methods are not entirely satisfactory because the required phosphonium halide compounds are relatively expensive and produce undesirable by-products which are difficult to remove from the 1,4-diaryl-2-fluoro-1,3-butadiene compounds. Accordingly, a need exists in the art for an improved process for the preparation of 1,4-diaryl-2-fluoro-1,3-butadiene compounds which avoids the use of phosphonium halide compounds.

It is, therefore, an object of the present invention to provide new compounds which are useful in the preparation of 1,4-diaryl-2-fluoro-1,3-butadiene compounds.

It is also an object of the present invention to provide an improved process for the preparation of 1,4-diaryl-2-fluoro-1,3-butadiene compounds which avoids the use of phosphonium halide compounds.

It is a further object of this invention to provide an improved process for the preparation of 1,4-diaryl-2-fluoro-2-butene compounds.

Other objects and advantages of the present invention will be apparent to those skilled in the art from the description below and the appended claims.

## SUMMARY OF THE INVENTION

The present invention provides 1,4-diaryl-2-fluoro-1-buten-3-ol compounds of the structural formula I

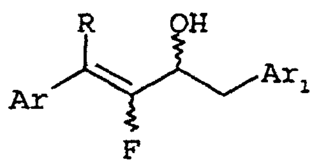

(I)

wherein

R is hydrogen, $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
phenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
phenoxypyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylpyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzoylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl,

$C_1$-$C_4$haloalkyl, $C_1$-$C_4$alkoxy or $C_1$-$C_4$haloalkoxy groups, or

a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups.

The present invention also provides a new process for the preparation of 1,4-diaryl-2-fluoro-1,3-butadiene compounds of the structural formula II

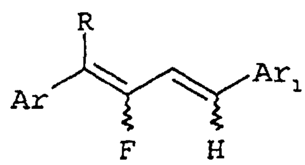

(II)

wherein

R is hydrogen, $C_1$-$C_4$alkyl, $C_1$-$C_4$haloalkyl, $C_3$-$C_6$cycloalkyl or $C_3$-$C_6$halocycloalkyl;

Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,

1- or 2-naphthyl optionally substituted with any combination of from one to three halogen, $C_1$-$C_4$alkyl, $C_1$-$C_4$haloalkyl $C_1$-$C_4$alkoxy or $C_1$-$C_4$haloalkoxy groups, or

a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and

$Ar_1$ is phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,

phenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,

biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,

phenoxypyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,

benzylpyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,

benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,

benzoylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,

1- or 2-naphthyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups, or

a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups, which process comprises reacting a 1,4-diaryl-2-fluoro-1-buten-3-ol compound of the structural formula I

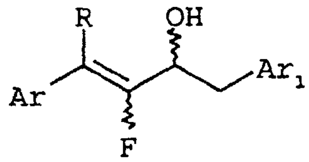

(I)

wherein Ar, $Ar_1$ and R are as hereinbefore defined with a sulfonyl chloride or sulfonic acid anhydride compound and a base.

The present invention further provides a new process for the preparation of 1,4-diaryl-2-fluoro-2-butene compounds having the structural formula III

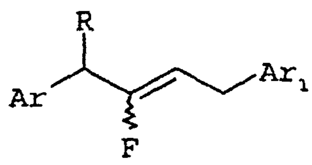

(III)

wherein

R is hydrogen, $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
phenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
phenoxypyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylpyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzoylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,

which process comprises:

(a) reacting a 1,4-diaryl-2-fluoro-1-buten-3-ol compound of the structural formula I

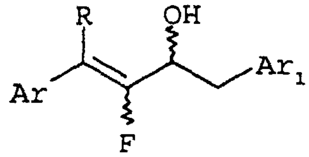

(I)

wherein Ar, $Ar_1$ and R are as described above with a sulfonyl chloride or sulfonic acid anhydride compound and a base to form a 1,4-diaryl-2-fluoro-1,3-butadiene compound of the structural formula II

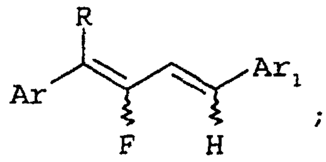

(II)

and

(b) reacting the 1,4-diaryl-2-fluoro-1,3-butadiene compound with: (1) an alkaline earth metal in the presence of a protic solvent, or (2) an alkali metal in the presence of an aprotic solvent.

## DETAILED DESCRIPTION OF THE INVENTION

The present invention provides 1,4-diaryl-2-fluoro-1-buten-3-ol compounds having the structural formula I

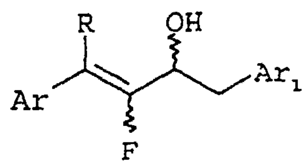

(I)

wherein Ar, $Ar_1$ and R are as described hereinbefore defined.

Preferred formula I compounds of this invention are those wherein

R is $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
3-biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups, or
3-benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups.

More preferred 1,4-diaryl-2-fluoro-1-buten-3-ol compounds of this invention are those wherein

R is isopropyl or cyclopropyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups.

1-(*p*-Chlorophenyl)-1-cyclopropyl-2-fluoro-4-(4-fluoro-3-phenoxyphenyl)-1-buten-3-ol and 1-(α,α,α-trifluoro-*p*-tolyl)-1-cyclopropyl-2-fluoro-4-(4-fluoro-3-phenoxyphenyl)-1-buten-3-ol are especially useful for the preparation of highly active insecticidal and acaricidal agents of formula III.

In formulas I, II and III above, the 5- and 6-membered heteroaromatic ring may suitably be a ring containing one to four heteroatoms selected from N, O and *S*, wherein the heteroatoms may be the same or different, e.g. the rings include, but are not limited to, pyridyl, pyrazolyl, imidazolyl, triazolyl, isoxazolyl, tetrazolyl, pyrazinyl, pyridazinyl, triazinyl, furanyl, thienyl and thiazolyl rings each optionally substituted as described in formulas I, II and III above.

Exemplary of "halogen" hereinabove are fluorine, chlorine, bromine and iodine. The terms "$C_1$-$C_4$haloalkyl", "$C_3$-$C_6$halocycloalkyl" and "$C_1$-$C_4$haloalkoxy" are defined as a $C_1$-$C_4$alkyl group, a $C_3$-$C_6$cycloalkyl group and a $C_1$-$C_4$alkoxy group substituted with one or more halogen atoms, respectively, wherein the halogen atoms may be the same or different.

When used herein as a group or part of a group, the term "alkyl" includes straight or branched chain alkyl groups such as methyl, ethyl, *n*-propyl, isopropyl, *n*-butyl, isobutyl, *s*-butyl and *t*-butyl. When used herein as a group or part of a group, the term "cycloalkyl includes cyclopropyl, cyclobutyl, cyclopentyl and cyclohexyl.

Groups containing two or more rings, such as phenoxyphenyl, phenoxypyridyl, biphenyl and benzylphenyl, which may be substituted, may be substituted on either ring unless otherwise specified herein.

In a preferred embodiment of the present invention, the 1,4-diaryl-2-fluoro-1,3-butadiene compounds of formula II are prepared by reacting a 1,4-diaryl-2-fluoro-1-buten-3-ol compound of formula I with a sulfonyl chloride or sulfonic acid anhydride compound and a base, preferably at a temperature ranging from about -78°C to 120°C, more preferably from about 20°C to 80°C, in the presence of a solvent.

In another preferred embodiment of the present invention, the 1,4-diaryl-2-fluoro-2-butene compounds of formula III are prepared by reacting a 1,4-diaryl-2-fluoro-1-buten-3-ol compound of formula I with a sulfonyl chloride or sulfonic acid anhydride compound and a base, preferably at a temperature ranging from about -78°C to 120°C, more preferably from about 20°C to 80°C, in the presence of a solvent to form a 1,4-diaryl-2-fluoro-1,3-butadienecompound of formula II, and reacting the formula II butadiene compound with an alkaline earth metal in the presence of a protic solvent.

Advantageously, the present invention provides a process for the preparation of 1,4-diaryl-2-fluoro-1,3-butadiene compounds which avoids the use of phosphonium halide compounds.

The product formula II and III compounds may be isolated by diluting the reaction mixture with water and extracting the product with a suitable extraction solvent. In the isolation procedure, conventional extraction solvents such as diethyl ether, ethyl acetate, toluene, methylene chloride, and the like, and mixtures thereof may be utilized.

Sulfonyl chloride compounds suitable for use in the present invention include, but are not limited to, unsubstituted and substituted phenylsulfonyl chlorides such as *p*-toluenesulfonyl chloride and the like, $C_1$-$C_6$alkylsulfonyl chlorides such as methanesulfonyl chloride and the like, and $C_1$-$C_6$haloalkylsulfonyl chlorides such as trifluoromethanesulfonyl chloride and the like. Sulfonic acid anhydrides suitable for use in this invention include, but are not limited to, unsubstituted and substituted phenylsulfonic acid anhydrides such as *p*-toluenesulfonic acid anhydride and the like, $C_1$-$C_6$alkylsulfonic acid anhydrides such as methanesulfonic acid anhydride and the like, and $C_1$-$C_6$haloalkylsulfonic acid anhydrides such as trifluoromethanesulfonic acid anhydride and the like. Bases suitable for use in the present invention include, but are not limited to, alkali metal hydrides such as sodium hydride, potassium hydride, lithium hydride and the like, alkaline earth metal hydrides such as calcium hydride and the like, alkali metal $C_1$-$C_6$alkoxides such as sodium methoxide, potassium *t*-butoxide and the like, $C_1$-$C_6$alkyllithiums such as *n*-butyllithium, *sec*-butyllithium, methyllithium and the like, and lithium dialkylamides such as lithium diisopropylamide, lithium isopropylcyclohexylamide and the like. Preferred bases include alkali metal hydrides.

Solvents suitable for use in the preparation of the formula II and III compounds of this invention include, but are not limited to, ethers such as tetrahydrofuran, dioxane, pyran, diethyl ether, 1,2-dimethoxyethane and the like; carboxylic acid amides such as N,N-dimethylformamide, N,N-dimethylacetamide and the like; dialkyl sulfoxides such as dimethyl sulfoxide and the like; nitriles such as acetonitrile, propionitrile and the like; aromatic hydrocarbons such as toluene, benzene, xylenes, mesitylene and the like; and halogenated aromatic hydrocarbons such as chlorobenzene, fluorobenzene and the like; and mixtures thereof. Preferred solvents for use in the preparation of the formula II and III compounds include ethers.

Protic solvents suitable for use in this invention include, but are not limited to, $C_1$-$C_6$alcohols such as methanol, ethanol and the like. Preferred protic solvents include methanol and ethanol. Aprotic solvents include, but are not limited to, ammonia; and ethers such as diethyl ether, tetrahydrofuran, dioxane, 1,2-dimethoxyethane and the like.

Alkaline earth metals suitable for use in the preparation of the formula III compounds include, but are not limited to, magnesium and calcium with magnesium being preferred. Alkali metals include, but are not limited to, lithium, sodium and potassium.

Preferred formula II and III compounds which may be prepared by the processes of this invention are those wherein

R is $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
3-biphenyl optionally substituted with any combination of from one to five halogen, $C_1$-$C_4$alkyl,

$C_1$-$C_4$haloalkyl, $C_1$-$C_4$alkoxy or $C_1$-$C_4$haloalkoxy groups, or

3-benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups.

More preferred 1,4-diaryl-2-fluoro-1,3-butadiene and 1,4-diaryl-2-fluoro-2-butene compounds which may be prepared by the processes of this invention are those
wherein

R is isopropyl or cyclopropyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups.

The present invention is especially useful for the preparation of 1-(*p*-chlorophenyl)-1-cyclopropyl-2-fluoro-4-(4-fluoro-3-phenoxyphenyl)-1,3-butadiene;
1-(α,α,α-trifluoro-*p*-tolyl)-1-cyclopropyl-2-fluoro-4-(4-fluoro-3-phenoxyphenyl)-1,3-butadiene;
1-[1-(α,α,α-trifluoro-*p*-tolyl)-2-fluoro-4-(4-fluoro-3-phenoxyphenyl)-2-butenyl]cyclopropane; and
1-[1-(*p*-chlorophenyl)-2-fluoro-4-(4-fluoro-3-phenoxyphenyl)-2-butenyl]cyclopropane.

The present invention further provides a process for the preparation of 1,4-diaryl-2-fluoro-1-buten-3-ol compounds of formula I which process comprises:

(a) reacting an arylmethanebromide compound of the structural formula IV

$$Ar_1CH_2Br$$

(IV)

wherein $Ar_1$ is as described hereinabove with a lithium $C_1$-$C_6$alkyltellurolate compound to form an intermediate compound of the structural formula V

$$Ar_1CH_2TeLi\ ;$$

(V)

(b) reacting the formula V intermediate compound with a $C_1$-$C_6$alkyllithium compound to form an intermediate compound of the structural formula VI

$$Ar_1CH_2Li\ ;$$

(VI)

and
(c) reacting the formula VI intermediate compound with a 3-aryl-2-fluoropropenal compound of the structural formula VII

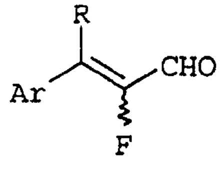

(VII)

wherein R and Ar are as hereinbefore defined.

In a preferred embodiment of the present invention, the 1,4-diaryl-2-fluoro-1-buten-3-ol compounds of formula I are prepared by reacting an arylmethanebromide compound of formula IV with a lithium $C_1$-$C_6$alkyltellurolate compound, preferably at a temperature ranging from about -78°C to about 30°C, more preferably from about -78°C to about 0°C, in the presence of a solvent to form an intermediate compound of formula V, reacting the formula V intermediate compound *in situ* with a $C_1$-$C_6$alkyllithium compound, preferably at a temperature ranging from about -78°C to about 30°C, more preferably from about -78°C to 0°C, to form an intermediate compound having the structural formula VI, and reacting the formula VI intermediate compound *in situ* with a 3-aryl-2-fluoropropenal compound of formula VII, preferably at a temperature ranging from about -78°C to about 30°C.

Lithium $C_1$-$C_6$alkyltellurolate compounds suitable for use in the present invention include, but are not limited to, lithium *n*-butyltellurolate, lithium *sec*-butyltellurolate, lithium *t*-butyltellurolate, lithium *n*-propyltellurolate and the like. $C_1$-$C_6$alkyllithium compounds suitable for use include, but are not limited to, *n*-butyllithium, *sec*-butyllithium, *n*-propyllithium, methyllithium and the like.

Solvents useful in the preparation of the formula I compounds of this invention include, but are not limited to, ethers such as diethyl ether, tetrahydrofuran, 1,2-dimethoxyethane and the like and mixtures thereof, with tetrahydrofuran being preferred.

In order to facilitate a further understanding of this invention, the following examples are presented primarily for the purpose of illustrating more specific details thereof. The scope of the invention should not be deemed limited by the examples but encompasses the entire subject matter defined in the claims.

**EXAMPLE 1**

**Preparation of 1-(*p*-Chlorophenyl)-1-cyclopropyl-2-fluoro-4-(4-fluoro-3-phenoxyphenyl)-1-buten-3-ol**

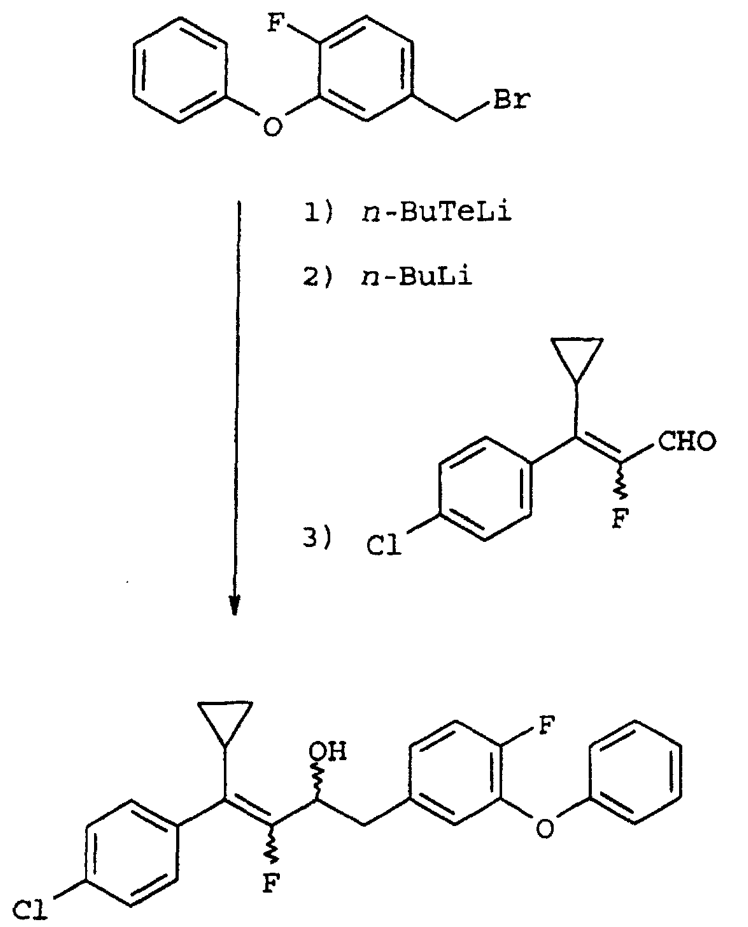


A solution of lithium *n*-butyltellurolate (10.5 mmol, *n*-BuTeLi), generated *in situ* from *n*-butyllithium (4.2 ml of 2.5 M solution in hexane, 10.5 mmol) and Te powder (1.34 g, 10.5 mmol) in tetrahydrofuran (10 ml) at 0°C, is treated with a solution of 3-phenoxy-4-fluorobenzyl bromide (2.81 g, 10 mmol) in tetrahydrofuran (10 ml) at 0°C, stirred for 30 minutes, cooled to -78°C, treated with a solution of *n*-butyllithium (4.2 ml of 2.5 M solution in hexane, 10.5 mmol), stirred for 30 minutes at -78°C, treated with a solution of *p*-chloro-β-cyclopropyl-α-fluorocinnamaldehyde (2.24 g, 10 mmol) in tetrahydrofuran (5 ml), warmed to room temperature with stirring for 3 hours, quenched with 2 N hydrochloric acid, and extracted with ethyl acetate. The combined organic extracts are washed with water, dried over anhydrous sodium sulfate and concentrated *in vacuo* to obtain a residue. Flash chromatography of the residue on silica gel using a 1:4 ethyl acetate/hexanes solution gives the title product (3.5 g, 82% yield) which is identified by $^{1}H$, $^{19}F$ and $^{13}C$ NMR spectral analyses.

Following essentially the same procedure, but using *p*-(trifluoromethyl)-β-cyclopropyl-α-fluorocinnamaldehyde, 1-(α,α,α-trifluoro-p-tolyl)-1-cyclopropyl-2-fluoro-4-(4-fluoro-3-phenoxyphenyl)-1-buten-3-ol is obtained.

**EXAMPLE 2**

**Preparation of 1-(*p*-Chlorophenyl)-1-cyclopropyl-2-fluoro-4-(4-fluoro-3-phenoxyphenyl)-1,3-butadiene**

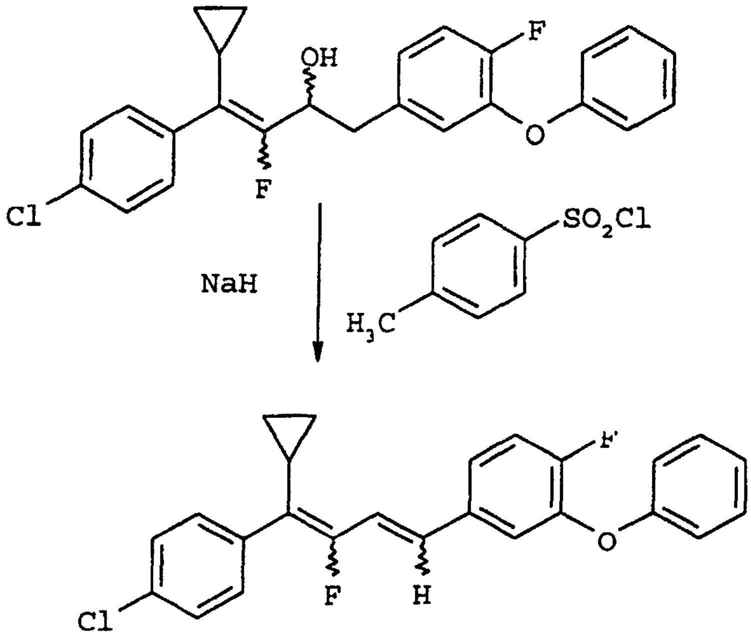


A suspension of sodium hydride (6.3 mg, 0.26 mmol) in tetrahydrofuran (1 ml) is treated with a solution of 1-(*p*-chlorophenyl)-1-cyclopropyl-2-fluoro-4-(4-fluoro-3-phenoxyphenyl)-1-buten-3-ol (106.7 mg, 0.25 mmol) in tetrahydrofuran (1.5 ml), stirred at 50°C for 10 minutes, cooled to room temperature, treated with a solution of *p*-toluenesulfonyl chloride (49.6 mg, 0.26 mmol) in tetrahydrofuran (1 ml), stirred at 60 °C for 1 hour, quenched with water, and extracted with ethyl acetate. The combined organic extracts are washed sequentially with water, saturated sodium hydrogen carbonate solution and water, dried over anhydrous sodium sulfate, and concentrated *in vacuo* to obtain a residue. Flash chromatography of the residue using silica gel and a 1:9 ethyl acetate/hexanes solution gives the title product (62 mg, 61% yield) which is identified by $^{1}H$, $^{19}F$ and $^{13}C$ NMR spectral analyses.

Following essentially the same procedure, but using 1-(α,α,α-trifluoro-*p*-tolyl)-1-cyclopropyl-2-fluoro-4-(4-fluoro-3-phenoxyphenyl)-1-buten-3-ol, 1-(α,α,α-trifluoro-*p*-tolyl)-1-cyclopropyl-2-fluoro-4-(4-fluoro-3-phenoxyphenyl)-1,3-butadiene is obtained.

## Claims

**1.** A compound of the structural formula I

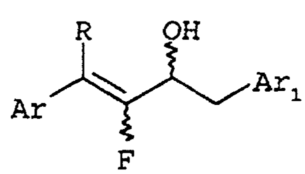

(I)

wherein

R is hydrogen, $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
phenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
phenoxypyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylpyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzoylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups.

**2.** The compound according to claim 1 wherein

R is $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
3-biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups, or
3-benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups.

**3.** The compound according to claim 2 wherein

R is isopropyl or cyclopropyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups.

**4.** The compound according to claim 1 selected from the group consisting of
1-(*p*-chlorophenyl)-1-cyclopropyl-2-fluoro-4-(4-fluoro-3-phenoxyphenyl)-1-buten-3-ol and
1-($\alpha$,$\alpha$,$\alpha$-trifluoro-*p*-tolyl)-1-cyclopropyl-2-fluoro-4-(4-fluoro-3-phenoxyphenyl)-1-buten-3-ol.

**5.** A process for the preparation of a 1,4-diaryl-2-fluoro-1,3-butadiene compound of the structural formula II

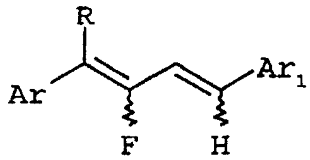

(II)

wherein

R is hydrogen, $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
phenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
phenoxypyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylpyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylphenyl opticnally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzoylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,

which process comprises reacting a 1,4-diaryl-2-fluoro-1-buten-3-ol compound of the structural formula I

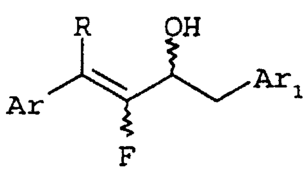

(I)

wherein Ar, $Ar_1$ and R are as hereinbefore defined with a sulfonyl chloride or sulfonic acid anhydride compound and a base.

**6.** The process according to claim 5 wherein the sulfonyl chloride is selected from the group consisting of phenylsulfonyl chloride, a substituted phenylsulfonyl chloride, a $C_1$-$C_6$alkylsulfonyl chloride and a $C_1$-$C_6$haloalkylsulfonyl

chloride; and the sulfonic acid anhydride is selected from the group consisting of phenylsulfonic acid anhydride, a substituted phenylsulfonic acid anhydride, a $C_1$-$C_6$alkylsulfonic acid anhydride and a $C_1$-$C_6$haloalkylsulfonic acid anhydride.

**7.** The process according to claim 5 wherein the base is selected from the group consisting of an alkali metal hydride, an alkaline earth metal hydride, an alkali metal $C_1$-$C_6$alkoxide, a $C_1$-$C_6$alkyllithium and a lithium dialkylamide.

**8.** The process according to claim 7 wherein the base is an alkali metal hydride.

**9.** The process according to claim 5 wherein the 1,4-diaryl-2-fluoro-1-buten-3-ol compound is reacted with the sulfonyl chloride or sulfonic acid anhydride compound and the base in the presence of a solvent.

**10.** The process according to claim 9 wherein the solvent is selected from the group consisting of an ether, a carboxylic acid amide, a dialkyl sulfoxide, a nitrite, an aromatic hydrocarbon and a halogenated aromatic hydrocarbon and mixtures thereof.

**11.** The process according to claim 5 wherein the 1,4-diaryl-2-ffuoro-1-buten-3-ol compound is reacted with the sulfonyl chloride or sulfonic acid anhydride compound and the base at a temperature ranging from about -78°C to about 120°C.

**12.** The process according to claim 5 wherein

R is $C_1$-$C_4$alkyl, $C_1$-$C_4$haloalkyl, $C_3$-$C_6$cycloalkyl or $C_3$-$C_6$halocycloalkyl ;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
3-biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups, or
3-benzylpheryl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups.

**13.** The process according to claim 12 wherein

R is isopropyl or cyclopropyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups.

**14.** The process according to claim 5 for the preparation of a compound selected from the group consisting of
1-(*p*-chlorophenyl)-1-cyclopropyl-2-fluoro-4-(4-fluoro-3-phenoxyphenyl)-1,3-butadiene and
1-(α,α,α-trifluoro-*p*-tolyl)-1-cyclopropyl-2-fluoro-4-(4-fluoro-3-phenoxyphenyl)-1,3-butadiene.

**15.** The process for the preparation of a 1,4-diaryl-2-fluoro-2-butene compound of the structural formula III

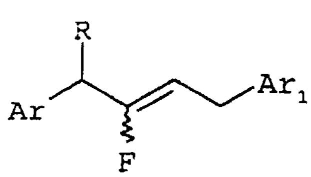

(III)

wherein

R is hydrogen, $C_1$-$C_4$alkyl, $C_1$-$C_4$haloalkyl, C3-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
phenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
phenoxypyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylpyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzoylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups, or
a 5- or 6-membered hetercaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,

which process comprises:

(a) reacting a 1,4-diaryl-2-fluoro-1-buten-3-ol compound of the structural formula I

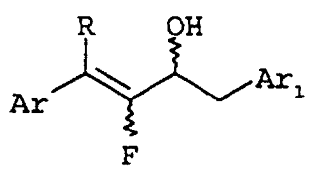

(I)

wherein Ar, $Ar_1$ and R are as described above with a sulfonyl chloride or sulfonic acid anhydride compound and a base to form a 1,4-diaryl-2-fluoro-1,3-butadiene compound of the structural formula II

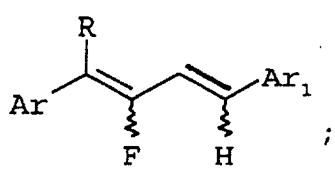

(II)

and

(b) reacting the 1,4-diaryl-2-fluoro-1,3-butadiene compound with: (1) an alkaline earth metal in the presence of a protic solvent, or (2) an alkali metal in the presence of an aprotic solvent.

**16.** The process according to claim 15 wherein the sulfonyl chloride is selected from the group consisting of phenylsulfonyl chloride, a substituted phenylsulfonyl chloride, a $C_1$-$C_6$alkylsulfonyl chloride and a $C_1$-$C_6$haloalkylsulfonyl chloride; and the sulfonic acid anhydride is selected from the group consisting of phenylsulfonic acid anhydride, a substituted phenylsulfonic acid anhydride, a $C_1$-$C_6$alkylsulfonic acid anhydride and a $C_1$-$C_6$haloalkylsulfonic acid anhydride.

**17.** The process according to claim 15 wherein the base is selected from the group consisting of an alkali metal hydride, an alkaline earth metal hydride, an alkali metal $C_1$-$C_6$alkoxide, a $C_1$-$C_6$alkyllithium and a lithium dialkylamide.

**18.** The process according to claim 15 wherein the 1,4-diaryl-2-fluoro-1-buten-3-ol compound is reacted with the sulfonyl chloride or sulfonic acid anhydride compound and the base in the presence of a solvent.

**19.** The process according to claim 18 wherein the solvent is selected from the group consisting of an ether, a carboxylic acid amide, a dialkyl sulfoxide, a nitrite, an aromatic hydrocarbon and a halogenated aromatic hydrocarbon and mixtures thereof.

**20.** The process according to claim 15 wherein the 1,4-diaryl-2-fluoro-1-buten-3-ol compound is reacted with the sulfonyl chloride or sulfonic acid anhydride compound and the base at a temperature ranging from about -78°C to about 120°C.

**21.** The process according to claim 15 wherein the 1,4-diaryl-2-fluoro-1,3-butadiene compound is reacted with the alkaline earth metal in the presence of the protic solvent.

**22.** The process according to claim 21 wherein the alkaline earth metal is magnesium.

**23.** The process according to claim 21 wherein the protic solvent is a $C_1$-$C_6$alcohol.

**24.** The process according to claim 15 wherein

R is $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
3-biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups, or
3-benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups.

**25.** The process according to claim 24 wherein

R is isopropyl or cyclopropyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups.

**26.** The process according to claim 15 for the preparation of a compound selected from the group consisting of
1-[1-(*p*-chlorophenyl)-2-fluoro-4-(4-fluoro-2-phenoxyphenyl)-2-butenyl]cyclopropane and
1-[1-(α,α,α-trifluoro-*p*-tolyl)-2-fluoro-4-(4-fluoro-3-phenoxyphenyl)-2-butenyl]cyclopropane.

**27.** A process for the preparation of a 1,4-diaryl-2-fluoro-1-buten-3-of compound of the structural formula I

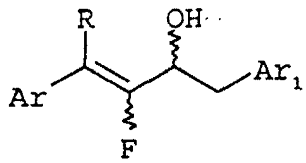

(I)

wherein

R is hydrogen, $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
phenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
phenoxypyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylpyridyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
benzoylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups,
1- or 2-naphthyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups, or
a 5- or 6-membered heteroaromatic ring optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups,

which process comprises:

(a) reacting an arylmethanebromide compound of the structural formula IV

$$Ar_1CH_2Br$$

(IV)

wherein $Ar_1$ is as described hereinabove with a lithium $C_1$-$C_6$alkyltellurolate compound to form an intermediate compound of the structural formula V

$$Ar_1CH_2TeLi \;;$$

(V)

(b) reacting the formula V intermediate compound with a $C_1$-$C_6$alkyllithium compound to form an intermediate compound of the structural formula VI

$Ar_1CH_2Li$ ;

(VI)

and

(c) reacting the formula VI intermediate compound with a 3-aryl-2-fluoropropenal compound of the structural formula VII

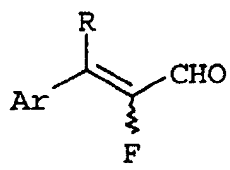

(VII)

wherein R and Ar are as hereinbefore defined.

**28.** The process according to claim 27 wherein the arylmethanebromide compound is reacted with the lithium $C_1$-$C_6$alkyltellurolate compound in the presence of a solvent to form the formula V intermediate compound, reacting the formula V intermediate compound *in situ* with the $C_1$-$C_6$alkyllithium compound to form the formula VI intermediate compound, and reacting the formula VI intermediate compound in situ with the 3-aryl-2-fluoropropenal compound.

**29.** The process according to claim 28 wherein the solvent is an ether.

**30.** The process according to claim 27 wherein

R is $C_1$-$C_4$alkyl, $C_1$-$C_4$haloalkyl, $C_3$-$C_6$cycloalkyl or $C_3$-$C_6$halocycloalkyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups,
3-biphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups, or
3-benzylphenyl optionally substituted with any combination of from one to five halogen, $C_1$-$C_4$alkyl, $C_1$-$C_4$haloalkyl, $C_1$-$C_4$alkoxy or $C_1$-$C_4$haloalkoxy groups.

**31.** The process according to claim 30 wherein

R is isopropyl or cyclopropyl;
Ar is phenyl optionally substituted with any combination of from one to three halogen, $C_1$-$C_4$alkyl, $C_1$-$C_4$haloalkyl, $C_1$-$C_4$alkoxy or $C_1$-$C_4$haloalkoxy groups; and
$Ar_1$ is 3-phenoxyphenyl optionally substituted with any combination of from one to six halogen, $C_1$-$C_4$alkyl, $C_1$-$C_4$haloalkyl, $C_1$-$C_4$alkoxy or $C_1$-$C_4$haloalkoxy groups.

**32.** The process according to claim 27 for the preparation of a compound selected from the group consisting of
1-(*p*-chlorophenyl)-1-cyclopropyl-2-fluoro-4-(4-fluoro-3-phenoxyphenyl)-1-buten-3-ol and
1-(α,α,α-trifluoro-*p*-tolyl)-1-cyclopropyl-2-fluoro-4-(4-fluoro-3-phenoxyphenyl)-1-buten-3-ol.

**Patentansprüche**

**1.** Verbindung der Strukturformel I

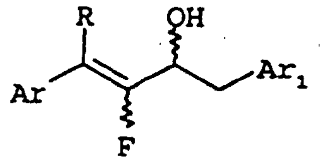

**(I)**

worin

R für Wasserstoff, $C_1$-$C_4$-Alkyl, $C_1$-$C_4$-Halogenalkyl, $C_3$-$C_6$-Cycloalkyl oder $C_3$-$C_6$-Halogencycloalkyl steht;

Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 1- oder 2-Naphthyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für einen 5- oder 6-gliedrigen heteroaromatischen Ring steht, der gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und

$Ar_1$ für Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Phenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Biphenyl steht, das gegebenenfalls durch eine beliebige Kombination von einem bis fünf Substituenten aus der Gruppe Halogen, $C_1$-$C_4$-Alkyl,

$C_1$-$C_4$-Halogenalkyl, $C_1$-$C_4$-Alkoxy oder $C_1$-$C_4$-Halogenalkoxy substituiert ist,
für Phenoxypyridyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzylpyridyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzoylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 1- oder 2-Naphthyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für einen 5- oder 6-gliedrigen heteroaromatischen Ring steht, der gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist.

**2.** Verbindung nach Anspruch 1, worin

R für $C_1$-$C_4$-Alkyl, $C_1$-$C_4$-Halogenalkyl, $C_3$-$C_6$-Cycloalkyl oder $C_3$-$C_6$-Halogencycloalkyl steht;

Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und

$Ar_1$ für 3-Phenoxyphenyl steht, das gegebenenfalls durch eine beliebige Kombination von einem bis sechs

Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 3-Biphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für 3-Benzylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist.

**3.** Verbindung nach Anspruch 2, worin

R für Isopropyl oder Cyclopropyl steht;
Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und
$Ar_1$ für 3-Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist.

**4.** Verbindung nach Anspruch 1, ausgewählt unter:

1-(p-Chlorphenyl)-1-cyclopropyl-2-fluor-4-(4-fluor-3-phenoxyphenyl)-1-buten-3-ol und
1-($\alpha$,$\alpha$,$\alpha$-Trifluor-p-tolyl)-1-cyclopropyl-2-fluor-4-(4-fluor-3-phenoxyphenyl)-1-buten-3-ol.

**5.** Verfahren zur Herstellung von 1,4-Diaryl-2-fluor-1,3-butadien-Verbindungen der Strukturformel II

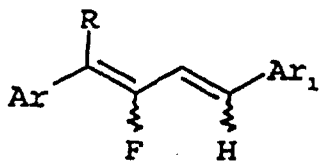

(II)

worin

R für Wasserstoff, $C_1$-$C_4$-Alkyl, $C_1$-$C_4$-Halogenalkyl, $C_3$-$C_6$-Cycloalkyl oder $C_3$-$C_6$-Halogencycloalkyl steht;
Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 1- oder 2-Naphthyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für einen 5- oder 6-gliedrigen heteroaromatischen Ring steht, der gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und
$Ar_1$ für Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Phenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Biphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Phenoxypyridyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzylpyridyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzoylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 1- oder 2-Naphthyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für einen 5- oder 6-gliedrigen heteroaromatischen Ring steht, der gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,

wobei bei dem Verfahren eine 1,4-Diaryl-2-fluor-1-buten-3-ol-Verbindung der Strukturformel I

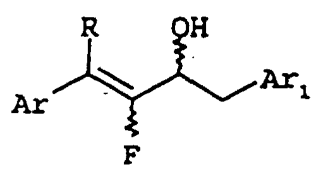

(I)

worin Ar, $Ar_1$ und R die zuvor genannten Bedeutungen aufweisen, mit einer Sulfonylchlorid- oder Sulfonsäureanhydrid-Verbindung und einer Base umgesetzt wird.

6. Verfahren nach Anspruch 5, wobei das Sulfonylchlorid unter Phenylsulfonylchlorid, substituierten Phenylsulfonylchloriden, $C_1$-$C_6$-Alkylsulfonylchloriden und $C_1$-$C_6$-Halogenalkylsulfonylchloriden ausgewählt ist; und das Sulfonsäureanhydrid unter Phenylsulfonsäureanhydrid, substituierten Phenylsulfonsäureanhydriden, $C_1$-$C_6$-Alkylsulfonsäureanhydriden und $C_1$-$C_6$-Halogenalkylsulfonsäureanhydriden ausgewählt ist.

7. Verfahren nach Anspruch 5, wobei die Base unter Alkalimetallhydriden, Erdalkalimetallhydriden, Alkalimetall-$C_1$-$C_6$-alkoxiden, $C_1$-$C_6$-Alkyllithium und Lithiumdialkylamiden ausgewählt ist.

8. Verfahren nach Anspruch 7, wobei es sich bei der Base um ein Alkalimetallhydrid handelt.

9. Vertahren nach Anspruch 5, wobei die Umsetzung der 1,4-Diaryl-2-fluor-1-buten-3-ol-Verbindung mit der Sulfonylchlorid- oder Sulfonsäureanhydrid-Verbindung und der Base in Gegenwart eines Lösungsmittels erfolgt.

10. Verfahren nach Anspruch 9, wobei das Lösungsmittel unter Ethem, Carbonsäureamiden, Dialkylsulfoxiden, Nitrilen, aromatischen Kohlenwasserstoffe und halogenierten aromatischen Kohlenwasserstoffen sowie Gemischen davon ausgewählt ist.

11. Verfahren nach Anspruch 5, wobei die 1,4-Diaryl-2-fluor-1-buten-3-ol-Verbindung bei einer Temperatur von etwa -78 °C bis etwa 120 °C mit der Sulfonylchlorid- oder Sulfonsäureanhydrid-Verbindung und der Base umgesetzt wird.

12. Verfahren nach Anspruch 5, wobei

R für $C_1$-$C_4$-Alkyl, $C_1$-$C_4$-Halogenalkyl, $C_3$-$C_6$-Cycloalkyl oder $C_3$-$C_6$-Halogencycloalkyl steht;

Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und

$Ar_1$ für 3-Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,

für 3-Biphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder

für 3-Benzylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist.

**13.** Verfahren nach Anspruch 12, wobei

R für Isopropyl oder Cyclopropyl steht;

Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und

$Ar_1$ für 3-Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist.

**14.** Verfahren nach Anspruch 5 zur Herstellung einer Verbindung ausgewählt unter:

1-(p-Chlorphenyl)-1-cyclopropyl-2-fluor-4-(4-fluor-3-phenoxyphenyl)-1,3-butadien und
1-(α,α,α-Trifluor-p-tolyl)-1-cyclopropyl-2-fluor-4-(4-fluor-3-phenoxyphenyl)-1,3-butadien.

**15.** Verfahren zur Herstellung von 1,4-Diaryl-2-fluor-2-buten-Verbindungen der Strukturformel III

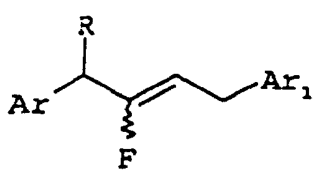

(III)

worin

R für Wasserstoff, $C_1$-$C_4$-Alkyl, $C_1$-$C_4$-Halogenalkyl, $C_3$-$C_6$-Cycloalkyl oder $C_3$-$C_6$-Halogencycloalkyl steht;

Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,

für 1- oder 2-Naphthyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder

für einen 5- oder 6-gliedrigen heteroaromatischen Ring steht, der gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und

$Ar_1$ für Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,

für Phenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy sub-

stituiert ist,
für Biphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Phenoxypyridyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzylpyridyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzoylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 1- oder 2-Naphthyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für einen 5- oder 6-gliedrigen heteroaromatischen Ring steht, der gegebenenfalis durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,

wobei bei dem Verfahren

a) eine 1,4-Diaryl-2-fluor-1-buten-3-ol-Verbindung der Strukturformel I

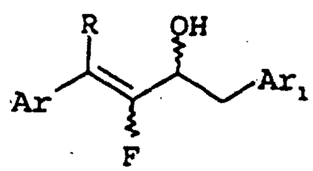

(I)

worin Ar, $Ar_1$ und R die zuvor genannten Bedeutungen aufweisen, mit einer Sulfonylchlorid- oder Sulfonsäureanhydrid-Verbindung und einer Base unter Bildung einer 1,4-Diaryl-2-fluor-1,3-butadien-Verbindung der Strukturformel II

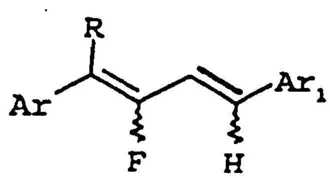

(II)

umgesetzt wird; und
b) die 1,4-Diaryl-2-fluor-1,3-butadien-Verbindung mit: (1) einem Erdalkalimetall in Gegenwart eines protischen Lösungsmittels oder (2) einem Alkalimetall in Gegenwart eines aprotischen Lösungsmittels umgesetzt wird.

**16.** Verfahren nach Anspruch 15, wobei das Sulfonylchlorid unter Phenylsulfonylchlorid, substituierten Phenylsulfonylchloriden, $C_1$-$C_6$-Alkylsulfonylchloriden und $C_1$-$C_6$-Halogenalkylsulfonylchloriden ausgewählt ist; und das Sulfonsäureanhydrid unter Phenylsulfonsäureanhydrid, substituierten Phenylsulfonsäureanhydriden, $C_1$-$C_6$-Alkylsulfonsäureanhydriden und $C_1$-$C_6$-Halogenalkylsulfonsäureanhydriden ausgewählt ist.

**17.** Verfahren nach Anspruch 15, wobei die Base unter Alkalimetallhydriden, Erdalkalimetallhydriden, Alkalimetall-$C_1$-$C_6$-alkoxiden, $C_1$-$C_6$-Alkyllithium und Lithiumdialkylamiden ausgewählt ist.

**18.** Verfahren nach Anspruch 15, wobei die 1,4-Diaryl-2-fluor-1-buten-3-ol-Verbindung in Gegenwart eines Lösungsmittels mit der Sufonylchlorid- oder Sulfonsäureanhydrid-Verbindung und der Base umgesetzt wird.

**19.** Verfahren nach Anspruch 18, wobei das Lösungsmittel unter Ethers, Carbonsäureamiden, Dialkylsulfoxiden, Nitrilen, aromatischen Kohlenwasserstoffen und halogenierten aromatischen Kohlenwasserstoffen sowie Gemischen davon ausgewählt ist.

**20.** Verfahren nach Anspruch 15, wobei die 1,4-Diaryl-2-fluor-1-buten-3-ol-Verbindung bei einer Temperatur von etwa -78 °C bis etwa 120 °C mit der Sulfonylchlorid- oder Sulfonsäureanhydrid-Verbindung und der Base umgesetzt wird.

**21.** Verfahren nach Anspruch 15, wobei die 1,4-Diaryl-2-fluor-1,3-butadien-Verbindung mit dem Erdalkalimetall in Gegenwart eines protischen Lösungsmittels umgesetzt wird.

**22.** Verfahren nach Anspruch 21, wobei es sich bei dem Erdalkalimetall um Magnesium handelt.

**23.** Verfahren nach Anspruch 21, wobei es sich bei dem protischen Lösungsmittel um einen $C_1$-$C_6$-Alkohol handelt.

**24.** Verfahren nach Anspruch 15, wobei

R für $C_1$-$C_4$-Alkyl, $C_1$-$C_4$-Halogenalkyl, $C_3$-$C_6$-Cycloalkyl oder $C_3$-$C_6$-Halogencycloalkyl steht;
Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und
$Ar_1$ für 3-Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 3-Biphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für 3-Benzylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substi-tuiert ist.

**25.** Verfahren nach Anspruch 24, wobei

R für Isopropyl oder Cyclopropyl steht;
Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und
$Ar_1$ für 3-Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist.

**26.** Verfahren nach Anspruch 15 zur Herstellung einer Verbindung, ausgewählt unter:

1-[1-p-Chlorphenyl)-2-fluor-4-(4-fluor-2-phenoxyphenyl)-2-butenyl]cyclopropan und
1-[1-(α,α,α-Trifluor-p-tolyl)-2-fluor-4-(4-fluor-3-phenoxyphenyl)-2-butenyl]cyclopropan.

**27.** Verfahren zur Herstellung von 1,4-Diaryl-2-fluor-1-buten-3-ol-Verbindungen der Strukturformel I

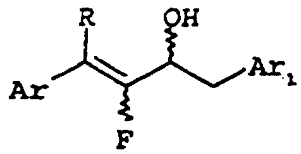

(I)

worin

R für Wasserstoff, $C_1$-$C_4$-Alkyl, $C_1$-$C_4$-Halogenalkyl, $C_3$-$C_6$-Cycloalkyl oder $C_3$-$C_6$-Halogencycloalkyl steht;

Ar für Phenyl steht, das gegebenenfalls durch eine beliebige Kombination von einem bis drei Substituenten aus der Gruppe Halogen; $C_1$-$C_4$-Alkyl; $C_1$-$C_4$-Halogenalkyl, $C_1$-$C_4$-Alkoxy oder $C_1$-$C_4$-Halogenalkoxy substituiert ist,
für 1- oder 2-Naphthyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für einen 5- oder 6-gliedrigen heteroaromatischen Ring steht, der gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und

$Ar_1$ für Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Phenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Biphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Phenoxypyridyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzylpyridyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für Benzoylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 1- oder 2-Naphthyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für einen 5- oder 6-gliedrigen heteroaromatischen Ring steht, der gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist;

wobei bei dem Verfahren

a) eine Arylmethanbromid-Verbindung der Strukturformel IV,

$$Ar_1CH_2Br$$

(IV)

worin $Ar_1$ die zuvor genannten Bedeutungen aufweist, mit einer Lithium-$C_1$-$C_6$-alkyltellurolat-Verbindung unter Bildung einer Zwischenverbindung der Strukturformel V

$$Ar_1CH_2TeLi$$

(V)

umgesetzt wird;

b) die Zwischenverbindung der Formel V mit einer $C_1$-$C_6$-Alkylltihium-Verbindung unter Bildung einer Zwischenverbindung der Strukturformel VI

$$Ar_1CH_2Li$$

(VI)

umgesetzt wird; und

c) die Zwischenverbindung der Formel VI mit einer 3-Aryl-2-fluorpropenal-Verbindung der Strukturformel VII

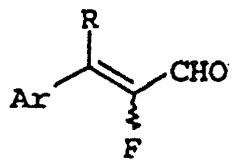

(VII)

worin R und Ar die zuvor genannten Bedeutungen aufweisen, umgesetzt wird.

**28.** Verfahren nach Anspruch 27, wobei die Umsetzung der Arylmethanbromid-Verbindung mit der Lithium-$C_1$-$C_6$-alkyltellurolat-Verbindung unter Bildung der Zwischenverbindung der Formel V in Gegenwart eines Lösungsmittels erfolgt, die Umsetzung der Zwischenverbindung der Formel V mit der $C_1$-$C_6$-Alkyllithium-Verbindung unter Bildung der Zwischenverbindung der Formel VI *in situ* erfolgt und die Umsetzung der Zwischenverbindung der Formel VI mit der 3-Aryl-2-fluorpropenal-Verbindung *in situ* erfolgt.

**29.** Verfahren nach Anspruch 28, wobei es sich bei dem Lösungsmittel um Ether handelt.

**30.** Verfahren nach Anspruch 27, wobei

R für $C_1$-$C_4$-Alkyl, $C_1$-$C_4$-Halogenalkyl, $C_3$-$C_6$-Cycloalkyl oder $C_3$-$C_6$-Halogencycloalkyl steht;

Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und

$Ar_1$ für 3-Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist,
für 3-Biphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist, oder
für 3-Benzylphenyl steht, das gegebenenfalls durch eine beliebige Kombination von einem bis fünf Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist.

**31.** Verfahren nach Anspruch 30, wobei

R für Isopropyl oder Cyclopropyl steht;
Ar für Phenyl steht, das gegebenenfalls durch eine beliebige Kombination von einem bis drei Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist; und
$Ar_1$ für 3-Phenoxyphenyl steht, das gegebenenfalls durch eine beliebige Kombination von einem bis sechs Substituenten aus der Gruppe Halogen, $C_1$-$C_4$-Alkyl, $C_1$-$C_4$-Halogenalkyl, $C_1$-$C_4$-Alkoxy oder $C_1$-$C_4$-Halogenalkoxy substituiert ist.

**32.** Verfahren nach Anspruch 27 zur Herstellung einer Verbindung, ausgewählt unter:

1-(p-Chlorphenyl)-1-cyclopropyl-2-fluor-4-(4-fluor-3-phenoxyphenyl)-1-buten-3-ol und
1-(α,α,α-Trifluor-p-tolyl)-1-cyclopropyl-2-fluor-4-(4-fluor-3-phenoxyphenyl)-1-buten-3-ol.

## Revendications

**1.** Composé répondant à la formule développée I

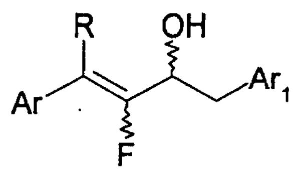

(I)

dans laquelle

R représente un atome d'hydrogène, un groupe alkyle en $C_1$-$C_4$, un groupe halogénoalkyle en $C_1$-$C_4$, un groupe cycloalkyle en $C_3$-$C_6$ ou un groupe halogénocycloalkyle en $C_3$-$C_6$ ;
Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe 1- ou 2-naphtyle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou
un cycle hétéroaromatique de 5 ou 6 chaînons facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, et
$Ar_1$ représente un groupe phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe phényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe biphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe phénoxypyridyle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe benzylpyridyle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe benzylphényle facultativement substitué par l'une quelconque des combinaisons de un à cinq

atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzoylephényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe 1- ou 2-naphtyle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou

un cycle hétéroaromatique de 5 ou 6 chaînons facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**2.** Composé selon la revendication 1, dans lequel R représente un groupe alkyle en $C_1$-$C_4$, un groupe halogénoalkyle en $C_1$-$C_4$, un groupe cycloalkyle en $C_3$-$C_6$ ou un groupe halogénocycloalkyle en $C_3$-$C_6$ ;

Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$ ; et

$Ar_1$ représente un groupe 3-phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe 3-biphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou

un groupe 3-benzylphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**3.** Composé selon la revendication 2, dans lequel

R représente un groupe isopropyle ou un groupe cyclopropyle ;
Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$; et
$Ar_1$ représente un groupe 3-phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**4.** Composé selon la revendication 1, choisi dans le groupe comprenant le 1-(p-chlorophényl)-1-cyclopropyl-2-fluoro-4-(4-fluoro-3-phénoxyphényl)-1-butén-3-ol et le 1-(α,α,α-trifluoro-p-tolyl)-1-cyclopropyl-2-fluoro-4-(4-fluoro-3-phénoxyphényl)-1-butén-3-ol.

**5.** Procédé pour la préparation d'un composé 1,4-diaryl-2-fluoro-1,3-butadiène répondant à la formule développée II

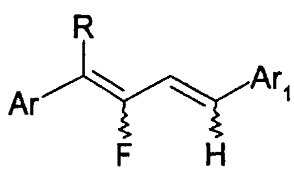

(II)

dans laquelle

R représente un atome d'hydrogène, un groupe alkyle en $C_1$-$C_4$, un groupe halogénoalkyle en $C_1$-$C_4$, un

groupe cycloalkyle en $C_3$-$C_6$ ou un groupe halogénocycloalkyle en $C_3$-$C_6$ ;

Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe 1- ou 2-naphtyle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou

un cycle hétéroaromatique de 5 ou 6 chaînons facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, et

$Ar_1$ représente un groupe phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe phényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe biphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe phénoxypyridyle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzylpyridyle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzylphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzoylephényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe 1- ou 2-naphtyle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou

un cycle hétéroaromatique de 5 ou 6 chaînons facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

lequel procédé comprend l'étape consistant à faire réagir un composé 1,4-diaryl-2-fluoro-1-butén-3-ol répondant à la formule développée I

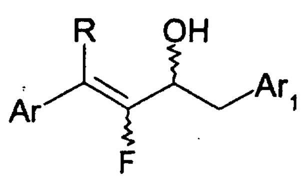

(I)

dans laquelle Ar, $Ar_1$ et R sont tels que définis ci-dessus avec un chlorure de sulfonyle ou un anhydride d'acide sulfonique et une base.

6. Procédé selon la revendication 5, dans lequel le chlorure de sulfonyle est choisi dans le groupe comprenant le chlorure de phénylsulfonyle, un chlorure de phénylsulfonyle substitué, un chlorure de (alkyle en $C_1$-$C_6$)sulfonyle et un chlorure de (halogénoalkyle en $C_1$-$C_6$)sulfonyle ; et l'anhydride d'acide sulfonique est choisi dans le groupe

comprenant l'anhydride d'acide phénylsulfonique, un anhydride d'acide phénylsulfonique substitué, un anhydride d'acide (alkyle en $C_1$-$C_6$)sulfonique et un anhydride d'acide (halogénoalkyle en $C_1$-$C_6$)sulfonique.

**7.** Procédé selon la revendication 5, dans lequel la base est choisie dans le groupe comprenant un hydrure de métal alcalin, un hydrure de métal alcalino-terreux, un alcoxyde en $C_1$-$C_6$ de métal alcalin, un (alkyl $C_1$-$C_6$)lithium et un dialkylamidure de lithium.

**8.** Procédé selon la revendication 7, dans lequel la base est un hydrure de métal alcalin.

**9.** Procédé selon la revendication 5, dans lequel le composé 1,4-diaryl-2-fluoro-1-butén-3-ol est mis à réagir avec le chlorure de sulfonyle ou l'anhydride d'acide sulfonique et la base en présence d'un solvant.

**10.** Procédé selon la revendication 9, dans lequel le solvant est choisi dans le groupe comprenant un éther, un amide d'acide carboxylique, un sulfoxyde de dialkyle, un nitrile, un hydrocarbure aromatique et un hydrocarbure aromatique halogéné et des mélanges de ceux-ci.

**11.** Procédé selon la revendication 5, dans lequel le composé 1,4-diaryl-2-fluoro-1-butén-3-ol est mis à réagir avec le chlorure de sulfonyle ou l'anhydride d'acide sulfonique et la base à une température comprise dans la plage allant d'environ -78° C à environ 120° C.

**12.** Procédé selon la revendication 5, dans lequel R représente un groupe alkyle en $C_1$-$C_4$, un groupe halogénoalkyle en $C_1$-$C_4$, un groupe cycloalkyle en $C_3$-$C_6$ ou un groupe halogénocycloalkyle en $C_3$-$C_6$ ;

Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$ ; et
$Ar_1$ représente un groupe 3-phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe 3-biphényle facultativement substitué par. l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou
un groupe 3-benzylphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**13.** Procédé selon la revendication 12, dans lequel

R représente un groupe isopropyle ou un groupe cyclopropyle;
Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$ ; et
$Ar_1$ représente un groupe 3-phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**14.** Composé selon la revendication 5, pour la préparation d'un composé choisi dans le groupe comprenant le 1-(p-chlorophényl)-1-cyclopropyl-2-fluoro-4-(4-fluoro-3-phénoxyphényl)-1,3-butadiène et le 1-(α,α,α-trifluoro-p-tolyl)-1-cyclopropyl-2-fluoro-4-(4-fluoro-3-phénoxyphényl)-1,3-butadiène.

**15.** Procédé pour la préparation d'un composé 1,4-diaryl-2-fluoro-2-butène répondant à la formule développée III

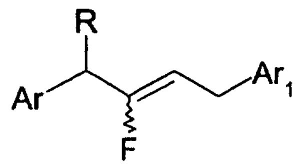

(III)

dans laquelle

R représente un atome d'hydrogène, un groupe alkyle en $C_1$-$C_4$, un groupe halogénoalkyle en $C_1$-$C_4$, un groupe cycloalkyle en $C_3$-$C_6$ ou un groupe halogénocycloalkyle en $C_3$-$C_6$ ;

Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe 1- ou 2-naphtyle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou

un cycle hétéroaromatique de 5 ou 6 chaînons facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, et

$Ar_1$ représente un groupe phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe phényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe biphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe phénoxypyridyle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzylpyridyle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzylphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzoylephényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe 1- ou 2-naphtyle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou

un cycle hétéroaromatique de 5 ou 6 chaînons facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, lequel procédé comprend les étapes consistant à :

(a) faire réagir un composé 1,4-diaryl-2-fluoro-1-butén-3-ol répondant à la formule développée I

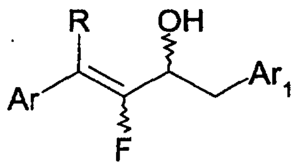

(I)

dans laquelle Ar, $Ar_1$ et R sont tels que définis ci-dessus avec un chlorure de sulfonyle ou un anhydride d'acide sulfonique et une base pour former un composé 1,4-diaryl-2-fluoro-1,3-butadiène de formule développée II

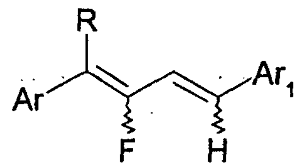

;

(II)

et

(b) faire réagir le composé 1,4-diaryl-2-fluoro-1,3-butadiène avec : (1) un métal alcalino-terreux en présence d'un solvant protique, ou (2) un métal alcalin en présence d'un solvant aprotique.

**16.** Procédé selon la revendication 15, dans lequel le chlorure de sulfonyle est choisi dans le groupe comprenant le chlorure de phénylsulfonyle, un chlorure de phénylsulfonyle substitué, un chlorure de (alkyle en $C_1$-$C_6$)sulfonyle et un chlorure de (halogénoalkyle en $C_1$-$C_6$)sulfonyle ; et l'anhydride d'acide sulfonique est choisi dans le groupe comprenant l'anhydride d'acide phénylsulfonique, un anhydride d'acide phénylsulfonique substitué, un anhydride d'acide (alkyle en $C_1$-$C_6$)sulfonique et un anhydride d'acide (halogénoalkyle en $C_1$-$C_6$)sulfonique.

**17.** Procédé selon la revendication 15, dans lequel la base est choisie dans le groupe comprenant un hydrure de métal alcalin, un hydrure de métal alcalino-terreux, un alcoxyde en $C_1$-$C_6$ d'un métal alcalin, un (alkyl $C_1$-$C_6$)lithium et un dialkylamidure de lithium.

**18.** Procédé selon la revendication 15, dans lequel le composé 1,4-diaryl-2-fluoro-1-butén-3-ol réagit avec le chlorure de sulfonyle ou l'anhydride d'acide sulfonique et la base en présence d'un solvant.

**19.** Procédé selon la revendication 18, dans lequel le solvant est choisi dans le groupe comprenant un éther, un amide d'acide carboxylique, un sulfoxyde de dialkyle, un nitrile, un hydrocarbure aromatique et un hydrocarbure aromatique halogéné et des mélanges de ceux-ci.

**20.** Procédé selon la revendication 15, dans lequel le composé 1,4-diaryl-2-fluoro-1-butén-3-ol réagit avec le chlorure de sulfonyle ou l'anhydride d'acide sulfonique et la base à une température comprise dans la plage allant d'environ -78° C à environ 120° C.

**21.** Procédé selon la revendication 15, dans lequel le composé 1,4-diaryl-2-fluoro-1,3-butadiène est mis à réagir avec le métal alcaline-terreux en présence d'un solvant protique.

**22.** Procédé selon la revendication 21, dans lequel le métal alcalino-terreux est le magnésium.

**23.** Procédé selon la revendication 21, dans lequel le solvant protique est un alcool en $C_1$-$C_6$.

**24.** Procédé selon la revendication 15, dans lequel

R représente un groupe alkyle en $C_1$-$C_4$, un groupe halogénoalkyle en $C_1$-$C_4$, un groupe cycloalkyle en $C_3$-$C_6$ ou un groupe halogénocycloalkyle en $C_3$-$C_6$ ;
Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$; et
$Ar_1$ représente un groupe 3-phénoxyphényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe 3-biphényle facultativement substitué par l'une quelconque des combinaisons de un à cinq atomes d'hatogène, groupes alkyles en $C_1$-$C_4$, halogénoalkyles en $C_1$-$C_4$, alcoxy en $C_1$-$C_4$ ou halogénoalcoxy en $C_1$-$C_4$, ou
un groupe 3-benzylphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**25.** Procédé selon la revendication 24, dans lequel

R représente un groupe isopropyle ou un groupe cyclopropyle ;
Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$ ; et
$Ar_1$ représente un groupe 3-phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**26.** Procédé selon la revendication 15, pour la préparation d'un composé choisi dans le groupe comprenant le 1-[1-(p-chlorophényl)-2-fluoro-4-(4-fluoro-2-phénoxyphényl)-2-butényl]cyclopropane et le 1-[1-(α,α,α-trifluoro-p-tolyl)-2-fluoro-4-(4-fluoro-3-phénoxyphényl) -2-butényl]cyclopropane.

**27.** Procédé pour la préparation d'un composé 1,4-diaryl-2-fluoro-1-butén-3-ol répondant à la formule développée I

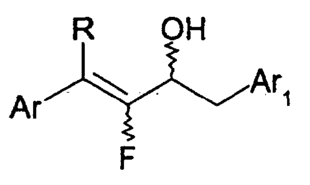

(I)

dans laquelle

R représente un atome d'hydrogène, un groupe alkyle en $C_1$-$C_4$, un groupe halogénoalkyle en $C_1$-$C_4$, un groupe cycloalkyle en $C_3$-$C_6$ ou un- groupe halogénocycloalkyle en $C_3$-$C_6$ ;
Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe 1- ou 2-naphtyle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou
un cycle hétéroaromatique de 5 ou 6 chaînons facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, et
$Ar_1$ représente un groupe phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe phényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe biphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe phénoxypyridyle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzylpyridyle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzylphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,

un groupe benzoylephényle facultativement substitué par l'une quelconque des combinaisons de un à cinq atomes d'halogéne, groupes alkyles en $C_1$-$C_4$, halogénoalkyles en $C_1$-$C_4$, alcoxy en $C_1$-$C_4$ ou halogénoalcoxy en $C_1$-$C_4$,

un groupe 1- ou 2-naphtyle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou

un cycle hétéroaromatique de 5 ou 6 chaînons facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
lequel procédé comprend les étapes consistant à:

(a) faire réagir un composé arylméthanebromure répondant à la formule développée IV

$$Ar_1CH_2Br$$

(IV)

dans laquelle $Ar_1$ est tel que défini ci-dessus avec un composé (alkyle en $C_1$-$C_6$)tellurolate de lithium pour former un composé intermédiaire répondant à la formule développée V

$$Ar_1CH_2TeLi\ ;$$

(V)

(b) faire réagir le composé intermédiaire répondant à la formule V avec un composé (alkyle en $C_1$-$C_6$)lithium pour former un composé intermédiaire répondant à la formule développée VI

$$Ar_1CH_2Li$$

(VI)

et
(c) faire réagir le composé intermédiaire répondant à la formule VI avec un composé 3-aryl-2-fluoropropénal répondant à la formule développée VII

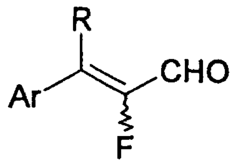

(VII)

dans laquelle R et Ar sont tels que définis ci-dessus.

**28.** Procédé selon la revendication 27, dans lequel le composé arylméthanebromure réagit avec le composé (alkyle en $C_1$-$C_6$)tellurolate de lithium en présence d'un solvant pour former le composé intermédiaire répondant à la formule V, en faisant réagir le composé intermédiaire répondant à la formule V *in situ* avec le composé (alkyle en $C_1$-$C_6$)lithium pour former le composé intermédiaire répondant à la formule VI, et en faisant réagir le composé intermédiaire répondant à la formule VI *in situ* avec le composé 3-aryl-2-fluoropropénal.

**29.** Procédé selon la revendication 28, dans lequel le solvant est un éther.

**30.** Procédé selon la revendication 27, dans lequel R représente un groupe alkyle en $C_1$-$C_4$, un groupe halogénoalkyle en $C_1$-$C_4$, un groupe cycloalkyle en $C_3$-$C_6$ ou un groupe halogénocycloalkyle en $C_3$-$C_8$ ;

Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$ ; et
$Ar_1$ représente un groupe 3-phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$,
un groupe 3-biphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$, ou
un groupe 3-benzylphényle facultativement substitué par l'une quelconque des combinaisons de un à cinq atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**31.** Procédé selon la revendication 30, dans lequel

R représente un groupe isopropyle ou un groupe cyclopropyle ;
Ar représente un groupe phényle facultativement substitué par l'une quelconque des combinaisons de un à trois atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$ ; et
$Ar_1$ représente un groupe 3-phénoxyphényle facultativement substitué par l'une quelconque des combinaisons de un à six atomes d'halogène, groupes alkyles en $C_1$-$C_4$, halogénoalkyles en $C_1$-$C_4$, alcoxy en $C_1$-$C_4$ ou halogénoalcoxy en $C_1$-$C_4$.

**32.** Procédé selon la revendication 27, pour la préparation d'un composé choisi dans le groupe comprenant le 1-(p-chlorophényl)-1-cyclopropyl-2-fluoro-4-(4-fluoro-3-phénoxyphényl)-1-butén-3-ol et le 1-(α,α,α-trifluoro-p-tolyl)-1-cyclopropyl-2-fluoro-4-(4-fluoro-3-phénoxyphényl)-1-butén-3-ol.